# EUROPEAN PATENT SPECIFICATION

(11) **EP 2 817 619 B1**
(45) Date of publication and mention of the grant of the patent: **25.07.2018**
(21) Application number: 13752111.8
(22) Date of filing: 22.02.2013
(51) Int. Cl.: A61K 39/395, A61P 35/00, G01N 33/50, G01N 33/574, C07K 16/28

(54) **PREDICTION OF RESPONSIVENESS TO TREATMENT WITH IMMUNOMODULATORY THERAPEUTICS AND METHOD OF MONITORING ABSCOPAL EFFECTS DURING SUCH TREATMENT**
VORHERSAGE DES ANSPRECHENS AUF EINE BEHANDLUNG MIT IMMUNMODULATORISCHEN THERAPEUTIKA UND VERFAHREN ZUR ÜBERWACHUNG DES ABSKOPAL-EFFEKTS IN DIESEM VERFAHREN
PRÉDICTION DE LA SENSIBILITÉ À UN TRAITEMENT PAR DES PRODUITS THÉRAPEUTIQUES D'IMMUNOMODULATION ET PROCÉDÉ DE SURVEILLANCE DES EFFETS ASCOPAUX AU COURS D'UN TEL TRAITEMENT

(30) Priority: 23.02.2012 US 201261602190 P
(43) Date of publication of application: 31.12.2014
(73) Proprietor: Sloan Kettering Institute For Cancer Research, New York, NY 10065 (US)
(72) Inventor: LESOKHIN, Alexander, M., New York, New York 10044 (US); WOLCHOK, Jedd, D., New York, New York 10016 (US)
(74) Representative: Chapman IP
(86) International application number: PCT/US2013/027475
(87) International publication number: WO 2013/126809

(56) References cited:
- WO-A2-2009/148915
- WO-A2-2012/149416
- Dennis A. Gastineau ET AL: "Association of an increased frequency of CD14posHLA-DR lo/neg monocytes with decreased time to progression in chronic lymphocytic leukaemia (CLL)", British Journal of Haematology,2012, vol. 156 3 November 2011 (2011-11-03), pages 674-676, XP055210972, Retrieved from the Internet: URL:http://www.ncbi.nlm.nih.gov/pmc/articl es/PMC3433277/pdf/nihms-401358.pdf [retrieved on 2015-09-03]
- CHRISTIANE MEYER ET AL: "Frequencies of circulating MDSC correlate with clinical outcome of melanoma patients treated with ipilimumab", CANCER IMMUNOLOGY, IMMUNOTHERAPY, vol. 63, no. 3, 1 March 2014 (2014-03-01), pages 247-257, XP055166338, ISSN: 0340-7004, DOI: 10.1007/s00262-013-1508-5
- YI LIN ET AL.: 'Immunosuppressive CD14+HLA-DRlow/- monocytes in B-cell non- Hodgkin Lymphoma.' BLOOD vol. 117, no. 3, 2011, pages 872 - 881, XP055081803
- GEOFFREY Y. KU ET AL.: 'Single-Institution Experience With Ipilimumab in Advanced Melanoma Patients in the Compassionate Use Setting.' CANCER vol. 116, no. 7, 2010, pages 1767 - 1775, XP055081802
- POUST J. ET AL.: 'Targeting metastatic melanoma' AM J HEALTH SYST PHARM vol. 65, no. 24 SUP, 2008, pages S9 - S15, XP008174490 & DATABASE PUBMED XP055082823 Database accession no. 19052265
- EVAN J LIPSON ET AL.: 'Ipilimumab: an Anti-CTLA-4 Antibody for Metastatic Melanoma.' CLIN CANCER RES 07 September 2011, page 1, 7, XP055081799

## Description

The present application relates to a method for predicting the efficacy of ipilimumab for enhancing anti-tumour immunity in a patient.

### Background of the Invention

Ipilimumab, an antibody that blocks the function of the immune inhibitory molecule cytotoxic T lymphocyte antigen 4 (CTLA-4), significantly prolongs survival in patients with metastatic melanoma. However, only 30% of patients derive clinical benefit from therapy. Therefore, defining biomarkers that could enable selection of patients more likely to respond to ipilimumab therapy is relevant for both practicing clinicians and for clinical trial design.

In addition, combinations of ipilimumab with chemotherapy, targeted therapy, other immunotherapy, and radiotherapy are being evaluated in an effort to increase the number of patients that respond. Pharmacodynamic markers that enable one to follow the immune effects of therapy may enhance other tools available for clinical decision-making.

The combination of ipilimumab and radiotherapy can lead to an abscopal effect. The abscopal effect refers to a rare phenomenon of tumor regression at a site distant from the primary site of radiotherapy (RT). (Mole RH., The British Journal of Radiology 1953;26:234-41.) Localized RT has been shown to induce abscopal effects in several malignancies including melanoma, lymphoma, and renal cell carcinoma. (Kingsley DP. , The British Journal of Radiology 1975;48:863-6; Robin et al., Medical and Pediatric Oncology 1981;9:473-6; Wersall et al., Acta Oncol 2006;45:493-7.2-4.) The biology underlying this effect is not completely clear but it may be mediated by immunologic mechanisms. (Drake C., Molecular Determinants of Radiation Response; New York: Springer, 2011. 251-263.)

In a recent clinical trial of 51 patients with unresectable melanoma treated with ipilimumab 33% achieved clinical benefit. Clinical benefit correlated with a rise in absolute lymphocyte count (ALC) after 2 doses to above 1000/mL and with immunity to NY-ESO1.^{1,2} NY-ESO-1 is an antigen expressed in 30-40% of patients with advanced melanoma but not present in normal adult tissues except testicular germ cells and placenta. (Jungbluth et al., International Journal of Cancer/Journal International du Cancer 2001;92:856-60.) Ipilimumab (Bristol-Myers Squibb, Princeton, NJ) has been shown to enhance immunity to NY-ESO-1, and patients with pre-existing NY-ESO-1 antibodies have an increased likelihood of benefiting from ipilimumab. (Yuan et al. Proceedings of the National Academy of Sciences of the United States of America 2011; 108:16723-8).

The present invention provides an alternative marker that is predictive of therapeutic efficacy of ipilimumab that also allows provides an indicator of immune mediated tumour elimination as occurs in the context of an abscopal effect following radiation.

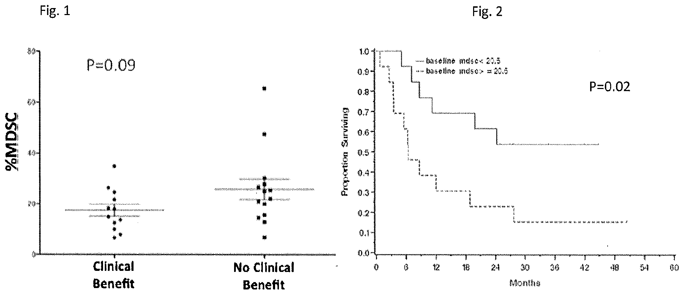

Dennis A. Gastineau et al in "Association of an increased frequency of CD14+HLA-DRlo/neg monocytes with decreased time to progression in chronic lymphocytic leukaemia (CLL)" British Journal of Haematology, 2012, vol 156 3 November 2011 (hereinafter referred to as D1) discloses at page 675, column 1, paragraph 3 to column 2, paragraph 1 (and Figure 1). Peripheral blood leukocytes in patients prior and following to therapy with alemtuzumab (CD52 inhibitor), rituximab (CD20 inhibitor) and GM-CSF were monitored on CD14+ and HLA-DR expression. D1 does not disclose the therapeutic being a CTLA4 inhibitor or more specifically being ipilimumab.

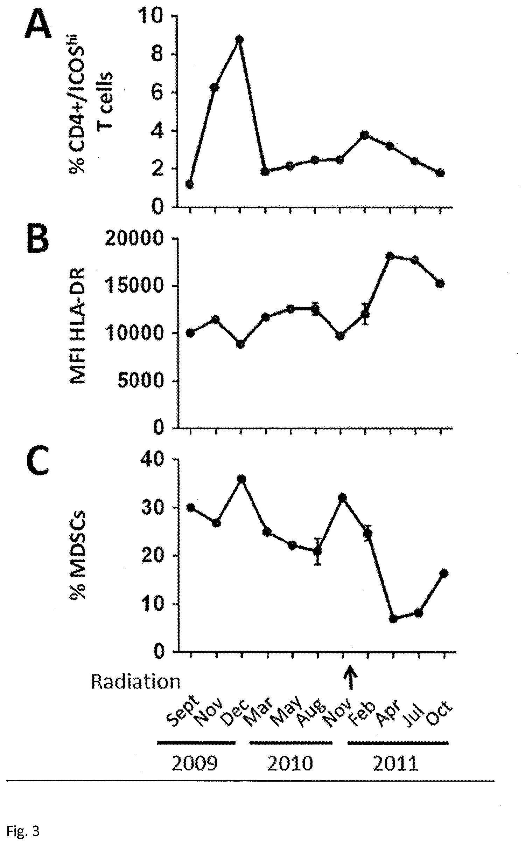

Yi Lin et al in "Immunosuppressive CD14+HLA-DRlo/neg monocytes in B-cell non-Hodgkin lymphoma (NHL)" Blood, Vol 117 , No 3 2011, pages 872-881 (hereinafter referred to as D4) describes isolation of immunophenotypes in B-cell NHL peripheral blood mononuclear cells and measurement of, inter alia, CD14 and HLA-DR expression. It was reported that patients with increased ratios of CD14⁺HLA-DR^{low-} monocytes had more aggressive disease and suppressed immune functions (abstract, Fig 3; page 875, left column, para. 3- page 876, right column, para. 1). However, larger patient populations are needed to determine the relationship of CD14⁺HLA-DR^{low-} monocytes with time to progression and overall survival (page 879, left column, para.2). D4 does not have any suggestion concerning the use of the population of monocytes for the efficacy of therapy, nor of the use of therapeutics such as CTLA4 inhibitors.

WO2009/148915 discloses (claims 1, 4 and 5) a method for predicting the likelihood a patient with cancer will have a favourable response to therapy with a co-stimulatory pathway modulator, comprising the steps of: (i) measuring absolute lymphocyte count (ALC) of patient samples collected over time subsequent, and optionally prior, to administration of said therapy; and (ii) calculating a slope of said absolute lymphocyte count, wherein patients that have a negative slope have a lower likelihood of achieving a favourable response to said therapy. The co-stimulatory pathway modulator can be a CTLA-4 antagonist such as ipilimumab. However WO2009/148915 D4 has no suggestion of CD14⁺HLA-DR^{low-} monocytes as measure to determine the effectiveness of the therapy.

### Summary of the Invention

The first aspect of the present invention provides a method for predicting efficacy of ipilimumab for enhancing anti-tumour immunity in a patient, the method according to the invention being as defined in the appended claims.

The method according to the first aspect of the present invention comprising the steps of:
measuring pre-treatment levels of peripheral blood monocytes that are CD14+ and HLA-DRlow (PBM14+HLA-DRlow) contained in a blood sample previously taken from the patient, comparing the measured pre-treatment levels in the blood sample to a discrimination threshold determined for a population of comparable patients, said discrimination threshold being selected to segregate the population into a sub-population of patients that tend to show a improved overall survival over a period of up to 2 years from ipilimumab treatment and a sub-population of patients that do not tend to show improved overall survival over a period of up to 2 years from ipilimumab treatment with a p value (statistical probability) of at most 0.10, and
determining that a measured level in the blood sample of PBM14+HLA-DRlow that is below the discrimination threshold indicates a likelihood of the efficacy of the therapeutic for enhancing said anti-tumour immunity in the patient.

In accordance with the method, a blood sample from the patient is tested for levels of monocytes having specific cell surface markers (CD14⁺, HLA-DR^{low}). Low levels of monocytes of this type (PBM14⁺HLA-DR^{low}) indicate a greater likelihood of therapeutic efficacy.

The method according to the first aspect of the present invention preferably further comprising
measuring pre-treatment absolute lymphocyte count (ALC) in the blood sample, comparing the measured ALC in the blood sample to an ALC-discrimination threshold determined for a population of comparable patients, said ALC-discrimination threshold being selected to segregate the population into a sub-population of patients that tend to show a clinical benefit from ipilimumab treatment and a sub-population of patients that do not tend to show a clinical benefit from ipilimumab treatment, and
determining that a measured ALC value greater than the ALC-discrimination threshold indicates a greater likelihood of the efficacy of the therapeutic for enhancing said anti-tumour immunity in the patient.

The method of the first aspect of the present invention, preferably further comprising measuring pre-treatment lactate dehydrogenase (LDH) level in the blood sample, comparing the determined LDH level to an LDH-discrimination threshold determined for a population of comparable patients, said LDH-discrimination threshold being selected to segregate the population into a sub-population of patients that tend to show a clinical benefit from ipilimumab treatment and a sub-population of patients that do not tend to show a clinical benefit from ipilimumab treatment, and
determining that a measured LDH level less than the LDH-discrimination threshold indicates a greater likelihood of the efficacy of the therapeutic for enhancing said anti-tumour immunity in the patient.

The second aspect of the present invention provides a method for assessing therapeutic progress of treatment for a patient who has been treated with ipilimumab as defined in the claims.

The method according to the second aspect of the present invention comprising the steps of
measuring pre-treatment levels of peripheral blood monocytes that are CD14+ and PBM14+HLA-DRlow in a sample taken from the patient before the treatment with the therapeutic and the radiation therapy,
measuring post-treatment levels of peripheral blood monocytes that are CD14+ and PBM14+HLA-DRlow in a sample taken from the patient after the treatment with the therapeutic and the radiation therapy, and
comparing the pre-treatment and post-treatment levels of PBM14+HLA-DRlow, wherein a decrease in the level of PBM14+HLA-DRlow indicates that therapeutic progress has commenced or is about to commence in the patient.

As indicated, the levels of PBM14⁺HLA-DR^{low} are determined after treatment of the patient with ipilimumab. Decreases in PBM14⁺HLA-DR^{low} indicate therapeutic progress.

Preferably, in the methods of both the first aspect and the second aspect, the patient is human.

### Detailed Description of the Invention

As is well known in the art, not all patients with a given disease benefit to the same extent for a given therapy, and in some cases some patients may exhibit no therapeutic benefit while others receive substantial benefit from the same treatment. As used in this application, the term "predicting the efficacy" means the making of a determination that a therapeutic is more likely to provide a therapeutically beneficial effect in the particular patient than in the population of patients suffering from the same disease as a whole. It does not require a certainty of therapeutic benefit or a demonstration of actual therapeutic benefit following treatment.

As used in this specification, the term "immunomodulatory leukocyte membrane protein" or "ILMP" refers to a membrane protein expressed in leukocytes that regulates immune response. The ILMP need not be expressed exclusively in leukocytes. In embodiments of the invention, the ILMP is a member of the immunoglobulin (Ig) superfamily or the tumour necrosis factor (TNF) superfamily.

Within the Ig superfamily, one specific example of an ILMP is CTLA4.
CTLA4 (Cytotoxic T-Lymphocyte Antigen 4) is also known as CD152 (Cluster of differentiation 152). Blocking CTLA4 with an antagonist, for example using antibodies against CTLA4 such as ipilimumab enhances immune response, and thus reduces immune system tolerance to tumours, thereby providing an immunotherapy strategy for patients with cancer.

Another example of an ILMP is Programmed Death 1, or PD-1, which is a member of the extended CD28/CTLA-4 family of T cell regulators and a member of the Ig superfamily. PD-1 is also known as CD279 and PDCD1. Zhang et al. shows that PD-1 and its ligand PD-L1 inhibit antitumor immune response in a murine acute myeloid leukemia model, and thus the importance of the PD-1/PD-L1 pathway in immune evasion by a hematologic malignancy.⁹ Furthermore, Curran et al, have shown that blockade of the PD-1/PD-L1 synergizes with CTLA-4 blockade in a murine melanoma model providing example of a combinatorial strategy involving both pathways of immune evasion¹.

Lymphocyte-activation gene 3 or LAG3 is another example of an ILMP that is a member of the Ig superfamily. LAG3 is also known as CD223. Triggering of LAG3 on peripheral CD8 T cells leads to peripheral tolerance and blockade of LAG3 with an antibody enhances CD8 dependent anti-tumor immunity.²

T cell immunoglobulin and mucin domain 3 or TIM3 is another example of an ILMP that is a member of the Ig superfamily. TIM3 is also known as KIM-3, TIMD3, and HAVcr-2. Blockade of TIM3 has been shown to suppress tumour outgrowth in a murine sarcoma model suggesting a role for this receptor in immune surveillance.³

Within the TNF superfamily, one specific example of an ILMP is 4-1BB, also known in humans as ILA (an acronym for induced by lymphocyte activation). 4-1 BB is also referred to as CD137 and TNFRSF9. Melero et al have used monoclonal antibodies against the 4-1BB antigen and demonstrate that administration of this antibody can eradicate established large poorly and highly immunogenic tumours in mice.⁴ Furthermore, Curran et al has shown that blockade of CTLA-4 can synergize with activation of 4-1BB providing another example where CTLA-4 blockade can lead to improved outcomes when combined with other immune based treatment approaches.⁵

Another example of an ILMP within the TNF superfamily is GITR (glucocorticoid induced tumour necrosis factor receptor family related gene), which in humans is often referred to as AITR (activation-inducible TNFR). Other names for GITR are TNFRSF18 and CD357. Therapeutics that enhance activation of GITR increase immune responses to melanocyte antigens and promote tumor rejection.⁹⁻¹¹

OX40 also known as CD134 or TNFRSF4 is another example of an ILMP within the TNF superfamily. In humans, the name given for this protein is commonly ACT35 (activation antigen-35). Therapeutic agents that target OX40 can lead to eradication of immunogenic tumors when used as a single agent and can be effectively combined with chemotherapy such as cyclophosphamide to eradicate less immunogenic tumors.^{6,7}

CD40 also known as Bp50, CDW40, P50, or TNFRSF5 is another example of an ILMP within the TNF superfamily. Therapeutic agents that send a positive signal through this receptor (agonist) alter the tumor stroma in favor of pancreatic cancer regression in mice and humans.¹⁴

Therapeutic agents targeting ILMP to which the present invention is applicable are those that interact specifically with the ILMP to enhance or stimulate the immune system. These therapeutics are commonly antibodies, including in particular monoclonal antibodies, the natural ligand (where the natural ligand results in immune system stimulation) or an immunostimulatory analog of the natural ligand. Table 1 lists specific agents that are known for various ILMP and the type of activity (agonist or antagonist) desired.

**Table 1**

| Target | Therapeutic | Agonist or Antagonist? | Reference |
|---|---|---|---|
| CTLA4 | ipilimumab | antagonist | ⁸ |
| CTLA4 | tremelimumab | antagonist | ClinicalTrials.gov Identifier: NCT00313794 |
| PD-1 | PD-L1 antibody (10F.9G2) was purchased from BioXCell | antagonist | ⁹ |
| PD-1 | CT-011 (Curetech) | antagonist | ¹⁰ |
| PD-1 | MDX-1106 (BMS) | Antagonist | ¹¹ |
| PD-1 | MK-3475 (Merck) | antagonist | ClinicalTrials.gov Identifier: NCT01295827 |
| LAG3 | Anti-CD223 (C9B7W) | antagonist | ² |
| TIM3 | Anti-TIM3 (RMT3-23) | antagonist | ³ |
| GITR/AITR | DTA-1 (provided by S. Sakaguchi) | Agonist | ^{12,13} |
| | | | ClinicalTrials.gov Identifier: NCT01239134 |
| | TRX518 | | |
| 4-1BB | PF-05082566 (Pfizer) | agonist | ClinicalTrials.gov |
| | | | Identifier: NCT01307267 |
| 4-1BB | BMS-663513 | agonist | ClinicalTrials.gov |
| | | | Identifier:NCT01471210 |
| OX40 | OX86 | agonist | ^{6,7} |
| CD40 | CP-870,893 (Pfizer) | agonist | ¹⁴ |

In accordance with an embodiment of the invention, a peripheral blood sample is evaluated to determine the amount of peripheral blood monocytes that are identified by the presence of CD14 on the cell surface and low levels of HLA-DR. These cells, referred to herein as PBM14⁺HLA-DR^{low} are a subset of myeloid derived suppressor cells (MDSC) which are identified by the present inventors to have relevance to the prediction of therapeutic efficacy. MDSCs generally are a mixed group of myeloid cells including immature granulocytes, macrophages, dendritic cells, and myeloid progenitors. The PBM14⁺HLA-DR^{low} subset of MDSCs was identified by Filipazzi et al. ¹⁵ as highly suppressive of lymphocyte functions, and significantly expanded in all metastatic melanoma patients, whereas they were undetectable in healthy donors.

Using the same type of analysis discussed below, additional markers that further identify a subset of MSC that is a predictor of therapeutic efficacy in other cancers may be found.

The determination that an individual patient is likely to be benefited by the treatment is based on a comparison between the amount of PBM14⁺HLA-DR^{low} in the sample, for example expressed as a % of the total number of cells and an empirically determined "discrimination threshold" which may vary depending on the species of the individual (for example human), the therapeutic and the type of cancer. One such determination is exemplified below in the context of specific experiment with metastatic melanoma being treated in humans with ipilimumab. In this case, the discrimination threshold was determined to be 20.5 % of the peripheral blood monocytes in the sample being PBM14⁺HLA-DR^{low}. This number may change with the development of larger data sets on which to base the statistical separation of data into the two groups (likely and not likely to be responsive) As a general matter, the discrimination threshold is selected such that a p value characterizing the statistical conclusion (probability) that overall survival over a period of up to 2 years in patients in whom the pre-treatment amount of PBM14⁺HLA-DR^{low} cells is higher than the threshold is statistically that same as (the null hypothesis) that of patients in whom the pre-treatment amount of PBM14⁺HLA-DR^{low} cells is lower than the threshold is at most 0.10, and is preferably less than 0.05. In the example shown below, p=0.002 when this comparison was made.

Lower quantity of PBM14⁺HLA-DR^{low} cells remained the strongest predictor of better survival even if other described predictors were taken into account. For example, if PBM14⁺HLA-DR^{low} cell quantity was corrected for absolute lymphocyte count at diagnosis or week 7 then PBM14⁺HLA-DR^{low} cell quantity still remained highly predictive (HR 1.10; 95% Cl 1.04, 1.17 p=0.0006). If PBM14⁺HLA-DR^{low} cell quantity was corrected for lactate dehydrogenase (LDH) then PBM14⁺HLA-DR^{low} cell quantity also remained highly predictive of overall survival (HR 1.06; 95% Cl 1.01, 1.11 p = 0.013, respectively). This suggests that PBM14⁺HLA-DR^{low} cell quantity is the strongest predictor of overall survival among patients being treated with an immunomodulatory therapeutic. Thus, the prediction ability is specific to the amount of not PBM14⁺HLA-DR^{low} cells. However, other indicators such as ALC or LDH levels can be used in combination as part of a combined prediction assay. Thus, the invention also provides a method comprising (in addition to the determination of PBM14⁺HLA-DR^{low} cells) determining pre-treatment absolute lymphocyte count (ALC) in the blood sample, and comparing the determined ALC to an ALC-discrimination threshold (for example 1000/mcl), wherein an ALC value greater than the ALC-discrimination threshold indicates a greater likelihood of therapeutic efficacy, and/or determining pre-treatment LDH level in the blood sample, and comparing the determined LDH level to an LDH-discrimination threshold (for example 276 U/I), wherein an LDH level less than the LDH-discrimination threshold indicates a greater likelihood of therapeutic efficacy. LDH is commonly evaluated as part of routine blood panels. It can be determined by monitoring enzyme activity through absorbance at 340 nm reflecting change in NADH concentration.

The method employed for determining the amount of PBM14⁺HLA-DR^{low} is not critical, and various methods can be employed. In the examples shown below, flow cytometry is employed, with known markers specific for CD14 and HLA-DR. The amount of PBM14⁺HLA-DR^{low} cells may also be determined by other techniques including immunohistochemical techniques and immunofluorescent cell counting.

The method of the invention is applicable to various cancer types that are treatable with ipilimumab, and is not limited to any particular cancer. For example, CTLA4 targeting therapeutic agents have applicability to melanoma, prostate cancer, and small cell and non-small cell lung cancer. In the case of PD-1 targeting therapeutics, a clinical trial is recruiting participants with acute myelogenous leukemia (AML) for treatment with a dendtritic cell AML vaccine and CT-011, an antibody that blockades PD-1. (Rosenblatt et al., J. Immunother. 2011 Jun;34(5):409-18.) CT-011 is also being studied in the context of other haematological malignancies such as diffuse large B cell lymphoma. Other specific cancers that may benefit from treatments with ipilimumab include breast cancer, colon cancer, renal cancer and myeloma.

In a further aspect of the invention, the inventors have also found that the same subset of MDSCs can be used to monitor the therapeutic progress of treatment with ipilimumab (a therapeutic that targets an ILMP) in combination with radiation therapy. In this case, testing for PBM14⁺HLA-DR^{low} after both the therapeutic and radiotherapy have been delivered to the patient, preferably 1 to 2 months after radiotherapy in order to predict or observe the onset of abscopal tumour reduction.

### Experimental results:

### Example 1

Peripheral blood from 26 patients with stage IV melanoma treated with ipilimumab 10mg/kg every 3 weeks for 4 doses as part of an expanded access program (BMS CA184-045) was assessed for the quantity (percentage) of CD14⁺, HLA-DR^{low/-} cells) pre-treatment, at week 7, week 12, and week 24 by flow cytometry. MDSC ability to inhibit T cell proliferation was tested using an *in vitro* suppression assay.

Absolute lymphocyte count (ALC) was measured pre-treatment and at week 7 by using a routine complete blood count. LDH levels were also measured pretreatment in the peripheral blood

We found that lower PBM14⁺HLA-DR^{low} quantity pre-treatment indicated clinical benefit measured at week 24 imaging (p=0.09) and predicted for improved overall survival (Hazard ratio 1.07 (1.03, 1.11) p=0.002). (Figs 1 and 2) As can be seen in Fig. 1, the values for PBM14⁺HLA-DR^{low} quantity exhibited some scatter, but values below 20.5% tended to show a clinical benefit and those with values above 20.5% tended not to. When this value was used as a discrimination threshold it provided a high correlation with overall survival as reflected in Fig. 2. Thus, pre-treatment PBM14⁺HLA-DR^{low} quantity predicts clinical response and overall survival following ipilimumab therapy.

The observed relationship was independent of pre-treatment or week 7 absolute lymphocyte counts (ALC) and pre-treatment LDH. If ALC and PBM14⁺HLA-DR^{low} quantity were evaluated together then the predictive value of PBM14⁺HLA-DR^{low} quantity remained (HR 1.10; 95% Cl 1.04, 1.17 p=0.0006). When LDH and PBM14⁺HLA-DR^{low} were analyzed together then PBM14⁺HLA-DR^{low} quantity was also still predictive of overall survival (HR 1.06; 95% Cl 1.01, 1.11 p = 0.013). Furthermore, a general trend of increasing PBM14⁺HLA-DR^{low} number by week 24 from the pre-treatment baseline was associated with patients that did not achieve clinical benefit. PBM14⁺HLA-DR^{low} suppressed peripheral blood T cell proliferation as measured by CFSE dilution in response to anti-CD3 antibody stimulation.

### Methods:(modified from ¹⁶)

Flow Cytometry: 5 x 10⁵ PBMCs from melanoma patients were washed with 2 ml FACS buffer (phosphate-buffered saline containing 2% bovine serum albumin and 0.05 *µ* M EDTA). The following antibodies were then added for 20 min at 4ºC: Lineage (CD3/CD16/CD19/CD20/CD56) cocktail FITC (special-ordered BD Pharmingen), CD14-PerCP Cy5.5, CD11b-APC Cy7, CD33-PE-Cy7 (BD Pharmingen), and HLA-DR-ECD (Beckman Coulter). Isotype controls included the appropriate fluorochrome conjugated mouse IgG₁, IgGₖ, IgG₂ₐ, or IgG_{2b k} (BD Pharmingen, Beckman Coulter, R&D Systems). The stained cells were detected using a CyAn flow cytometer. All analysis was performed using FlowJo software (Treestar, Ashland, OR).

Suppression Assay: 2 x 10⁵ CFSE-labeled CD14⁻PBMCs with or without CD14⁺ cells were cultured in 96-well flat-bottom α-CD3-specific Ab coated plates (OKT3, 100 mcl at 0.5µg/mL for 2 hours at 37 ºC) in RPMI 1640 medium supplemented with 10% FBS and IL-2 (10 IU/mL; Roche, Mannheim, Germany). After 5 days, cells were harvested, stained with CD3-PECy7, CD4-ECD, CD8-APCCy7 (BD Pharmingen) and CFSE signal of gated CD8⁺ T cells (CD3⁺ CD4⁻) was measured by flow cytometry.

### Example 2

A female patient was diagnosed with cutaneous melanoma in April, 2004 at age 33. Biopsy of a mole on her upper back revealed melanoma, non-ulcerated, with a Breslow thickness of 1.53mm. She underwent a wide local excision of her primary lesion along with a left axillary sentinel lymph node biopsy. There was no residual melanoma at the primary site, and the five axillary lymph nodes removed were not involved. She remained disease-free until 2008 when a routine chest-x-ray revealed a new 2.0cm pulmonary nodule in her left lower lobe. The nodule was hypermetabolic by positron emission tomography (PET) with a standard uptake value of 5.9. There were no additional sites of hypermetabolic foci. Cytology from a computed tomography (CT)- guided percutaneous biopsy of the pulmonary nodule revealed metastatic melanoma. Sequenom mass-spectrometry genotyping revealed no known mutations that affect the BRAF gene such as the BRAF V600E mutation.

Standard cisplatin, vinblastine, and temozolomide (CVT) chemotherapy was initiated, and after two cycles, a CT scan demonstrated stability of her pulmonary nodule and no evidence of additional metastases. The solitary pulmonary nodule was resected via a left, lower lobectomy in February 2009 with pathology confirming metastatic melanoma.

In August 2009, a surveillance CT scan detected recurrent disease with a new pleural based paraspinal mass and right hilar lymphadenopathy. In September 2009, she enrolled in a clinical trial at our institution, (CA-184-087, NCT00920907), a randomized, open-label trial to compare the safety and pharmacokinetics of ipilimumab manufactured by one of two distinct processes. She received ipilimumab at a dose of 10mg/kg every three weeks for four doses as part of induction therapy. A follow-up CT scan in December 2009 (12 weeks after ipilimumab initiation) demonstrated overall stable disease with slight enlargement of the pleural mass (not shown). Responses to ipilimumab are not always seen on the initial CT scan, 12-weeks after treatment initiation,12 and she was permitted to continue with ipilimumab as maintenance therapy given every 12 weeks.

Over the course of 2010, she had slight radiographic evidence of worsening disease but continued with maintenance ipilimumab since she was clinically well. She developed mild, asymptomatic hypothyroidism requiring thyroid hormone supplementation but had no other significant treatment-related toxicity. By November 2010, however, she had progressive enlargement of the pleural based paraspinal mass and new splenic lesions. To address right sided back pain caused by the paraspinal mass, palliative RT was initiated. In December 2010, she received 2850 cGy in 3 fractions over 7 days to the paraspinous mass with 6 MV photons using a coplanar 6-field intensity modulated image guided technique prescribed to the 100% isodose line encompassing the planning target volume, which is in the range of acceptable, commonly used dose fractionation schemes. In January 2011, one month after RT, a CT scan had not yet shown response at the primary irradiated site, right hilar lymph node, and spleen. She received one additional ipilimumab dose in February 2011. By April 2011, her targeted paraspinal lesion had regressed significantly (D). Remarkably, lesions in areas not targeted by RT had also regressed [right hilar lymphadenopathy and spleen]. A subsequent CT scan obtained in October 2011 (10 months after RT) showed stability with the continued presence of minimal disease .

In the only metastatic lesion available for analysis, a pulmonary nodule removed before ipilimumab treatment, NY-ESO-1 expression was confirmed by immunohistochemistry (IHC) showing homogeneous and strong positivity and by reverse transcription-polymerase chain reaction.

Using serum samples collected before the first ipilimumab treatment and before and after RT, we measured the antibody titers against whole NY-ESO-1 recombinant protein and against specific synthetic portions of the NY-ESO-1 protein by ELISA. Before all therapy, the patient was seropositive for whole NY-ESO-1 protein, with reactivity primarily confined to an epitope(s) contained within the N-terminal (amino acids 1-68) portion of the protein. During ipilimumab treatment, and in parallel with increasing disease burden, antibody titers to the whole NY-ESO-1 protein and to the N-terminal portion increased. Titers remained elevated, though trended lower, as her disease burden decreased.

After completing RT, the patient had a >30-fold increase in antibody titers to an epitope(s) within the central portion (amino acids 71-130), correlating with the time of disease resolution. Lastly, the patient had a seroconversion to an epitope(s) in the C-terminal portion (amino acids 119-180) during treatment with ipilimumab before RT, which corresponded to the time of increasing disease. Results were considered significant if the change in titers exceeded 5x between two time points. Seroreactivity to dihydrofolate reductase was used as a negative control.

Measurements of CD4+ T-cell activation based on inducible costimulator (ICOS) expression (CD4+/ICOShi) and myeloid lineage activation based upon MDSC quantity and HLA-DR expression on CD14+ monocytes (PBM14⁺HLA-DR^{low}) were performed by flow cytometry in peripheral blood mononuclear cells at serial time points coincident with measurements of NY-ESO-1 titers. Before RT, CD4+/ICOShi T cells (Figure 3A) increased during ipilimumab induction and then decreased. A clear trend in PBM14⁺HLA-DR^{low} quantity is not apparent during this time. Following RT, there was a second increase in CD4+/ICOShi cells and an increase in HLA-DR expression in CD14+ monocytes (Figure 3B) with a reciprocal, marked decline in the quantity of PBM14⁺HLA-DR^{low} that preceded radiographic disease regression, which was not evident until April 2011.

The patient demonstrated a systemic disease response following localized RT in the setting of prior disease progression on ipilimumab. The off-target right hilar lymph node mass and spleen received low, non-therapeutic doses of radiation (133cGy and 2.3cGy, respectively), further supporting that disease regression in these distant sites was due to an enhanced systemic response. Delayed responses occurring 18-20 weeks after ipilimumab are well known, yet in our opinion, the 19-month interval between the start of ipilimumab and disease response, in the context of the more recent RT, is more supportive of an abscopal effect.

RT has been shown to increase presentation of antigen by myeloid cells within the tumor stroma and thereby enhance T-cell killing of tumour cells. Analysis of the peripheral blood CD14+ cellular compartment revealed several signs of myeloid lineage activation including increased HLA-DR expression and a reduction in PBM14⁺HLA-DR^{low} quantity following RT. Since these changes preceded the reduction in tumour burden as determined by CT scan, they provide early signals of a shift in immune phenotype from immune escape toward immune mediated tumour elimination and thus are useful for monitoring the therapeutic progress/benefits being afforded the patient.

### References:

1. Curran MA, Montalvo W, Yagita H, Allison JP. PD-1 and CTLA-4 combination blockade expands infiltrating T cells and reduces regulatory T and myeloid cells within B16 melanoma tumors. Proc Natl Acad Sci U S A;107:4275-4280.
2. Grosso JF, Kelleher CC, Harris TJ, et al. LAG-3 regulates CD8+ T cell accumulation and effector function in murine self- and tumor-tolerance systems. J Clin Invest. 2007;117:3383-3392.
3. Ngiow SF, von Scheidt B, Akiba H, Yagita H, Teng MW, Smyth MJ. Anti-TIM3 antibody promotes T cell IFN-gamma-mediated antitumor immunity and suppresses established tumors. Cancer Res;71:3540-3551.
4. Melero I, Shuford WW, Newby SA, et al. Monoclonal antibodies against the 4-1BB T-cell activation molecule eradicate established tumors. Nat Med. 1997;3:682-685.
5. Curran MA, Kim M, Montalvo W, Al-Shamkhani A, Allison JP. Combination CTLA-4 blockade and 4-1BB activation enhances tumor rejection by increasing T-cell infiltration, proliferation, and cytokine production. PLoS One;6:e19499.
6. Hirschhorn-Cymerman D, Rizzuto GA, Merghoub T, et al. OX40 engagement and chemotherapy combination provides potent antitumor immunity with concomitant regulatory T cell apoptosis. J Exp Med. 2009;206:1103-1116.
7. Piconese S, Valzasina B, Colombo MP. OX40 triggering blocks suppression by regulatory T cells and facilitates tumor rejection. J Exp Med. 2008;205:825-839.
8. Hodi FS, O'Day SJ, McDermott DF, et al. Improved Survival with Ipilimumab in Patients with Metastatic Melanoma. N Engl J Med. 2010.
9. Zhang L, Gajewski TF, Kline J. PD-1/PD-L1 interactions inhibit antitumor immune responses in a murine acute myeloid leukemia model. Blood. 2009;114:1545-1552.
10. Rosenblatt J, Glotzbecker B, Mills H, et al. PD-1 blockade by CT-011, anti-PD-1 antibody, enhances ex vivo T-cell responses to autologous dendritic cell/myeloma fusion vaccine. J Immunother;34:409-418.
11. Brahmer JR, Drake CG, Wollner I, et al. Phase I study of single-agent anti-programmed death-1 (MDX-1106) in refractory solid tumors: safety, clinical activity, pharmacodynamics, and immunologic correlates. J Clin Oncol;28:3167-3175.
12. Cohen AD, Diab A, Perales MA, et al. Agonist anti-GITR antibody enhances vaccine-induced CD8(+) T-cell responses and tumor immunity. Cancer Res. 2006;66:4904-4912.
13. Cohen AD, Schaer DA, Liu C, et al. Agonist anti-GITR monoclonal antibody induces melanoma tumor immunity in mice by altering regulatory T cell stability and intra-tumor accumulation. PLoS One;5:e10436.
14. Beatty GL, Chiorean EG, Fishman MP, et al. CD40 agonists alter tumor stroma and show efficacy against pancreatic carcinoma in mice and humans. Science;331:1612-1616.
15. Filipazzi P, Valenti R, Huber V, et al. Identification of a new subset of myeloid suppressor cells in peripheral blood of melanoma patients with modulation by a granulocyte-macrophage colony-stimulation factor-based antitumor vaccine. J Clin Oncol. 2007;25:2546-2553.
16. Lesokhin AM, Hohl TM, Kitano S, et al. Monocytic CCR2+ Myeloid-Derived Suppressor Cells Promote Immune Escape by Limiting Activated CD8 T-cell Infiltration into the Tumor Microenvironment. Cancer Res. 2012;72:876-886.

## Claims

1. A method for predicting the efficacy of ipilimumab for enhancing anti-tumour immunity in a patient, the method comprising the steps of:
measuring pre-treatment levels of peripheral blood monocytes that are CD14⁺ and HLA-DR^{low}(PBM14⁺HLA-DR^{low}) contained in a blood sample previously taken from the patient,
comparing the measured pre-treatment levels in the blood sample to a discrimination threshold determined for a population of comparable patients, said discrimination threshold being selected to segregate the population into a sub-population of patients that tend to show improved overall survival over a period of up to 2 years from ipilimumab treatment and a sub-population of patients that do not tend to show improved overall survival over a period of up to 2 years from ipilimumab treatment with a p value (statistical probability) of at most 0.10, and
determining that a measured level in the blood sample of PBM14⁺HLA-DR^{low} that is below the discrimination threshold indicates a likelihood of the efficacy of the therapeutic for enhancing said anti-tumour immunity in the patient.

2. The method of claim 1, further comprising
measuring pre-treatment absolute lymphocyte count (ALC) in the blood sample, comparing the measured ALC in the blood sample to an ALC-discrimination threshold determined for a population of comparable patients, said ALC-discrimination threshold being selected to segregate the population into a sub-population of patients that tend to show improved overall survival over a period of up to 2 years from ipilimumab treatment and a sub-population of patients that do not tend to show improved overall survival over a period of up to 2 years from ipilimumab treatment, and
determining that a measured ALC value greater than the ALC-discrimination threshold indicates a greater likelihood of the efficacy of the therapeutic for enhancing said anti-tumour immunity in the patient.

3. The method of claim 1 or 2, further comprising
measuring pre-treatment lactate dehydrogenase (LDH) level in the blood sample, comparing the determined LDH level to an LDH-discrimination threshold determined for a population of comparable patients, said LDH-discrimination threshold being selected to segregate the population into a sub-population of patients that tend to show improved overall survival over a period of up to 2 years from ipilimumab treatment and a sub-population of patients that do not tend to show improved overall survival over a period of up to 2 years from ipilimumab treatment, and
determining that a measured LDH level less than the LDH-discrimination threshold indicates a greater likelihood of the efficacy of the therapeutic for enhancing said anti-tumour immunity in the patient.

4. A method for assessing therapeutic progress for a patient who has been treated with ipilimumab in combination with radiation therapy, the method comprising the steps of
measuring pre-treatment levels of peripheral blood monocytes that are PBM14⁺HLA-DR^{low} in a sample taken from the patient before the treatment with the therapeutic and the radiation therapy,
measuring post-treatment levels of peripheral blood monocytes that are PBM14⁺HLA-DR^{low} in a sample taken from the patient after the treatment with the therapeutic and the radiation therapy, and
comparing the pre-treatment and post-treatment levels of PBM14⁺HLA-DR^{low}, wherein a decrease in the level of PBM14⁺HLA-DR^{low} indicates that therapeutic progress has commenced or is about to commence in the patient.

5. The method of any of claims 1 to 4, wherein the patient is human.

## Patentansprüche

1. Ein Verfahren zur Vorhersage der Wirksamkeit von Ipilimumab zur Verstärkung der Anti-Tumor-Immunität bei einem Patienten, wobei das Verfahren die folgenden Schritte beinhaltet:
Messen der vor der Behandlung bestehenden Spiegel der Monozyten im peripheren Blut, die CD14⁺ und HLA-DR^{low} (PBM14⁺HLA-DR^{low}) sind, die in einer dem Patienten zuvor entnommenen Blutprobe enthalten sind,
Vergleichen der gemessenen vor der Behandlung bestehenden Spiegel in der Blutprobe mit einer für eine Population vergleichbarer Patienten ermittelten Ansprechschwelle, wobei die Ansprechschwelle so ausgewählt ist, dass sie die Population aufteilt, und zwar in eine Subpopulation von Patienten, die tendenziell ein verbessertes Gesamtüberleben über einen Zeitraum von bis zu 2 Jahren ab der Ipilimumab-Behandlung zeigen, und eine Subpopulation von Patienten, die tendenziell kein verbessertes Gesamtüberleben über einen Zeitraum von bis zu 2 Jahren ab der Ipilimumab-Behandlung zeigen, mit einem *p*-Wert (statistische Wahrscheinlichkeit) von höchstens 0,10, und
Bestimmen, dass ein gemessener Spiegel in der Blutprobe von PBM14⁺HLA-DR^{low} der unterhalb der Ansprechschwelle liegt, anzeigt, dass eine Wirksamkeit des Therapeutikums in Bezug auf die Verstärkung der Anti-Tumor-Immunität bei dem Patienten wahrscheinlich ist.

2. Verfahren gemäß Anspruch 1, das ferner Folgendes beinhaltet:
Messen der vor der Behandlung bestehenden absoluten Lymphozytenzahl (ALC) in der Blutprobe,
Vergleichen der gemessenen ALC in der Blutprobe mit einer für eine Population vergleichbarer Patienten bestimmten ALC-Ansprechschwelle, wobei die ALC-Ansprechschwelle so ausgewählt ist, dass sie die Population aufteilt, und zwar in eine Subpopulation von Patienten, die tendenziell ein verbessertes Gesamtüberleben über einen Zeitraum von bis zu 2 Jahren ab der Ipilimumab-Behandlung zeigen, und eine Subpopulation von Patienten, die tendenziell kein verbessertes Gesamtüberleben über einen Zeitraum von bis zu 2 Jahren ab der Ipilimumab-Behandlung zeigen, und
Bestimmen, dass ein gemessener ALC-Wert, der größer ist als die ALC-Ansprechschwelle, anzeigt, dass eine größere Wahrscheinlichkeit besteht, dass das Therapeutikum in Bezug auf die Verstärkung der Anti-Tumor-Immunität bei dem Patienten Wirksamkeit besitzt.

3. Verfahren gemäß Anspruch 1 oder 2, das Folgendes beinhaltet:
Messen des vor der Behandlung bestehenden Lactatdehydrogenase(LDH)-Spiegels in der Blutprobe,
Vergleichen des bestimmten LDH-Spiegels mit einer für eine Population vergleichbarer Patienten bestimmten LDH-Ansprechschwelle, wobei die LDH-Ansprechschwelle so ausgewählt ist, dass sie die Population aufteilt, und zwar in eine Subpopulation von Patienten, die tendenziell ein verbessertes Gesamtüberleben über einen Zeitraum von bis zu 2 Jahren ab der Ipilimumab-Behandlung zeigen, und eine Subpopulation von Patienten, die tendenziell kein verbessertes Gesamtüberleben über einen Zeitraum von bis zu 2 Jahren ab der Ipilimumab-Behandlung zeigen, und
Bestimmen, dass ein gemessener LDH-Spiegel, der geringer ist als die LDH-Ansprechschwelle, anzeigt, dass eine größere Wahrscheinlichkeit besteht, dass das Therapeutikum in Bezug auf die Verstärkung der Anti-Tumor-Immunität bei dem Patienten Wirksamkeit besitzt.

4. Verfahren zur Untersuchung des therapeutischen Fortschritts für einen Patienten, der mit Ipilimumab in Kombination mit Strahlentherapie behandelt wurde, wobei das Verfahren die folgenden Schritte beinhaltet:
Messen des Behandlung bestehenden Spiegels von Monozyten im peripheren Blut, die PBM14⁺HLA-DR^{low} sind, in einer dem Patienten vor der Behandlung mit dem Therapeutikum und der Strahlentherapie entnommenen Probe,
Messen der nach der Behandlung bestehenden Spiegel von Monozyten im peripheren Blut, die PBM14⁺HLA-DR^{low} sind, in einer dem Patienten nach der Behandlung mit dem Therapeutikum und der Strahlentherapie entnommenen Probe, und
Vergleichen der vor der Behandlung bestehenden und nach der Behandlung bestehenden Spiegel von PBM14⁺HLA-DR^{low}, wobei eine Senkung des Spiegels von PBM14⁺HLA-DR^{low} anzeigt, dass der therapeutische Fortschritt bei dem Pateinten begonnen hat oder im Begriff ist, zu beginnen.

5. Verfahren nach einem der Ansprüche 1 bis 4, wobei der Patient ein Mensch ist.

## Revendications

1. Procédé permettant de prédire l'efficacité de l'ipilimumab afin de renforcer l'immunité anti-tumorale chez un patient, le procédé comprenant les étapes suivantes :
la mesure du taux avant traitement de monocytes sanguins périphériques qui sont CD14⁺ et HLA-DR^{low} (PBM14⁺HLA-DR^{low}) contenus dans un échantillon sanguin prélevé sur le patient au préalable,
la comparaison du taux avant traitement mesuré dans l'échantillon sanguin à un seuil de discrimination déterminé pour une population de patients comparables, ledit seuil de discrimination étant choisi pour séparer la population en une sous-population de patients qui tend à présenter une meilleure survie globale sur une période allant jusqu'à 2 ans à partir du traitement par ipilimumab et une sous-population de patients qui ne tend pas à présenter une meilleure survie globale sur une période allant jusqu'à 2 ans à partir du traitement par ipilimumab avec une valeur p (probabilité statistique) de 0,10 au plus, et
la détermination du fait qu'un taux mesuré dans l'échantillon sanguin de PBM14⁺HLA-DR^{low} qui est inférieur au seuil de discrimination indique une probabilité de l'efficacité de l'agent thérapeutique pour renforcer ladite immunité anti-tumorale chez le patient.

2. Procédé de la revendication 1, comprenant en outre
la mesure de la numération absolue des lymphocytes (ALC) avant traitement dans l'échantillon sanguin,
la comparaison de l'ALC mesurée dans l'échantillon sanguin à un seuil de discrimination d'ALC déterminé pour une population de patients comparables, ledit seuil de discrimination d'ALC étant choisi pour séparer la population en une sous-population de patients qui tend à présenter une meilleure survie globale sur une période allant jusqu'à 2 ans à partir du traitement par ipilimumab et une sous-population de patients qui ne tend pas à présenter une meilleure survie globale sur une période allant jusqu'à 2 ans à partir du traitement par ipilimumab, et
la détermination du fait qu'une valeur d'ALC mesurée supérieure au seuil de discrimination d'ALC indique une plus grande probabilité de l'efficacité de l'agent thérapeutique pour renforcer ladite immunité anti-tumorale chez le patient.

3. Procédé de la revendication 1 ou 2, comprenant en outre
la mesure du taux de lactate déshydrogénase (LDH) avant traitement dans l'échantillon sanguin,
la comparaison du taux de LDH déterminé à un seuil de discrimination de LDH déterminé pour une population de patients comparables, ledit seuil de discrimination de LDH étant choisi pour séparer la population en une sous-population de patients qui tend à présenter une meilleure survie globale sur une période allant jusqu'à 2 ans à partir du traitement par ipilimumab et une sous-population de patients qui ne tend pas à présenter une meilleure survie globale sur une période allant jusqu'à 2 ans à partir du traitement par ipilimumab, et
la détermination du fait qu'un taux de LDH mesuré inférieur au seuil de discrimination de LDH indique une plus grande probabilité de l'efficacité de l'agent thérapeutique pour renforcer ladite immunité anti-tumorale chez le patient.

4. Procédé permettant d'évaluer la progression thérapeutique pour un patient ayant été traité par ipilimumab en association avec une radiothérapie, le procédé comprenant les étapes suivantes :
la mesure du taux avant traitement de monocytes sanguins périphériques qui sont PBM14⁺HLA-DR^{low} dans un échantillon prélevé sur le patient avant le traitement par l'agent thérapeutique et la radiothérapie,
la mesure du taux après traitement de monocytes sanguins périphériques qui sont PBM14⁺HLA-DR^{low} dans un échantillon prélevé sur le patient après le traitement par l'agent thérapeutique et la radiothérapie, et
la comparaison des taux avant traitement et après traitement de PBM14⁺HLA-DR^{low}, une diminution du taux de PBM14⁺HLA-DR^{low} indiquant que la progression thérapeutique a débuté ou est sur le point de débuter chez le patient.

5. Procédé de l'une quelconque des revendications 1 à 4, dans lequel le patient est un être humain.
